(19) Europäisches Patentamt
European Patent Office
Office européen des brevets

(11) Publication number: **0 436 379 A2**

(12) # EUROPEAN PATENT APPLICATION

(21) Application number: **90314244.6**

(22) Date of filing: **24.12.90**

(51) Int. Cl.5: **C07C 51/15**, C07C 65/03, C07C 65/05, C07C 65/11

(30) Priority: **27.12.89 KR 8919718**

(43) Date of publication of application: **10.07.91 Bulletin 91/28**

(84) Designated Contracting States: **DE FR GB**

(71) Applicant: **Kolon Industries Inc.**
**45, Mugyo-dong**
**Jung-gu Seoul(KR)**

(72) Inventor: **Choi, Tae Keun**
**803, Chungsil Apt.12, Taechi-dong**
**Kangnam-gu Seoul(KR)**
Inventor: **Park, Ho Jin**
**447-830, Sangin-dong Talseo-gu,**
**Taegu-si(KR)**
Inventor: **Kim, Ha Won,**
**401-402, Jookong Apt. Hyungkok 4, Hyungkok 119**
**Blok, Kumi-si Kyungsangbukdo(KR)**

(74) Representative: **Lawrence, Peter Robin Broughton et al**
**GILL JENNINGS & EVERY 53-64 Chancery Lane**
**London WC2A 1HN(GB)**

(54) **A process for preparation of an aromatic carboxylic acid.**

(57) The invention relates to a process for the preparation of an aromatic carboxylic acid or a derivative thereof, characterised by dissolving a derivative of a hydroxylated aromatic compound in an organic solvent, especially ethylenegylcol monoethyl ether, 3-methoxy-1-butanol or 1-methoxy-2-propanol, or mixtures thereof, in the presence pf $CO_2$ or a source of $CO_2$ in an autoclave and optionally neutralising the derivative of the carboxylic acid. The derivatives are preferably alkaline salts.

EP 0 436 379 A2

Xerox Copy Centre

## PROCESS FOR THE PREPARATION OF AN AROMATIC CARBOXYLIC ACID

The present invention relates to a process for the preparation of an aromatic carboxylic acid and more precisely, to the preparation of an aromatic carboxylic acid from a derivative of a hydroxylated aromatic compound.

Previously an aromatic carboxylic acid has been prepared by dissolving resorcinol and alkali in water, and passing $CO_2$ through the solution (Japan patent Laid-Open Pub. No, 59-184, 140), but this reaction has a low productivity (20%) and a low yield (50%) due to the equilibrium. An aromatic carboxylic acid may also be prepared by dissolving phenol and NaOH in water, distilling them to remove water and contacting the precipitate with $CO_2$ at a temperature between 220 and 250° C under 150 atm pressure, but method is very energy consuming, the yield is low (50%) and the use of such high pressure involves a risk. It is also known to prepare aromatic carboxylic acid by dissolving phenol and $Na_2CO_3$ in glycerol, and passing $CO_2$ through the solution. The disadvantage of this method is that the reaction time is long and the yield is low because sodium phenoxide is relatively insoluble in the solvent.

The present invention provides a method for making aromatic carboxylic acids which avoids the above mentioned problems. The method of the invention generally involves a shorter reaction time, and can give a yield of up to 90%.

Accordingly, the invention provides a process for the preparation of an aromatic carboxylic acid or a derivative thereof, which is characterised by dissolving a derivative of a hydroxylated aromatic compound in an organic solvent chosen from those represented by formula (I) or (II) or mixtures thereof in the presence of $CO_2$ or a source of $CO_2$ in an autoclave;

$$R - X - (CH_2)_n - Y \qquad (I)$$

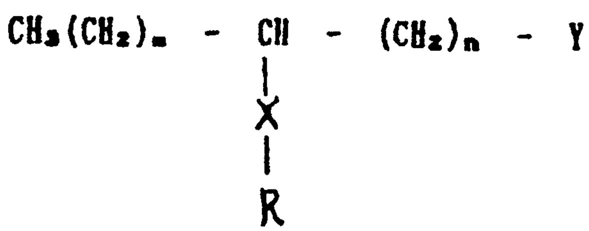

(II)

wherein, R is an alkyl group having one to 4 carbon atoms,
X is -O-. -NH-, -S-,or

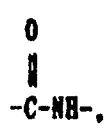

Y is -OH-, $-NH_2$ or

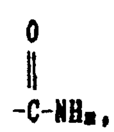

and
n is an integer from, 1 to 4,
m is an integer from 0 to 3.

This invention further provides a process for the preparation of an aromatic carboxylic acid characterized in that is comprises the steps of

(i) reacting an alkali salt of a hydroxylated aromatic compound in an organic solvent chosen from those

represented by formula (I) or (II) or mixtures thereof in an autoclave

$$R - X - (CH_2)n - Y \qquad (I)$$

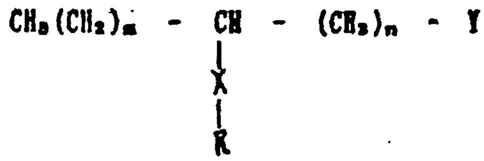 (II)

wherein R is an alkyl having one to 4 carbon atoms X is -O-, -NH-, -S-, or

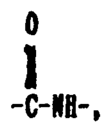

Y is -OH-, $-NH_2$ or

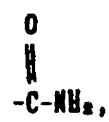

and
n is an integer from 1 to 4,
m is an integer from 0 to 3. to form an alkali salt of an aromatic carboxylic acid, and
(ii) neutralizing the alkali salt of the aromatic carboxylic acid to obtain the aromatic carboxylic acid.

Suitable hydroxylated aromatic compounds include phenol, resorcinol and naphthol. The derivatives of the hydroxylated aromatic compounds are preferably alkali salts such as hydroxides or carbonates.

The derivatives may be added to the solvent, or may preferably be prepared by adding salts of alkali metals to the organic solvent, together with the hydroxylated aromatic compound. Suitable alkali metal salts include for example NaOH, KOH, $Na_2CO_3$ $K_2CO_3$ , $kHCO_3$ and $NaHCO_3$. The carbonates or bicarbonates may provide some or all of the $CO_2$.

It is preferred that between 1.1 and 2.2 moles of alkali metal salt is added per mole of hydroxylate aromatic compound.

Preferred organic solvents include ethylene glycol monomethyl ether, 1-methoxy-2-propanol and 3-methoxy-1-butanol.

Suitable alkali metal salts of hydroxylated aromatic compound are the sodium salts of phenol, naphthol or resorcinol, which are easily dissolved in the organic solvent used in the process of the invention.

The process of the invention may suitably be carried out at a temperature of from 50 to 150°C, preferably from 90 to 140°C. The reaction may be performed at atmospheric pressure, but is preferably performed at a pressure of carbon dioxide of from 1 to 10 atmospheres pressure. The aromatic carboxylic acid is precipitated from the reaction mixture.

The surprising speed of the reaction is due to Le Chateliers equilibrium principle, due to the removal of carboxylic acid from the reaction mixture.

The advantage of the process according to the present invention is that the reaction time is considerably shortened, and a yield of over 90% and often much higher may be obtained. Yields approaching 100% may be obtained because only a small amount of the unreacted materials and the solvent are recovered after filtration.

Further details of the present invention are to be found in the following Examples.

Example 1

108.2g (1.15 mole) of phenol, 211.3g (1.53 mole) of potassium carbonate and 461 g of 1-methoxy-2-propanol (solvent 1) are added to a 1-liter autoclave, reacted together at a temperature of between 100 and 110° C for 4 hours maintaining 10 atm of $CO_2$, followed by being cooled at room temperature and filtered.

The filtered solids are dissolved in 300g of $H_2O$, neutralized by 160g of concentrated HCl and the resulting precipitate is filtered. The white solid thereby obtained is 118.3g (0.86 mole, yield 74.5%) of 4-hydroxy-benzoic acid, having a melting point of from 215 to 216° C.

Example 2

105.2g (0.96 mole) of resorcinol, 200.2g (1.45 mole) of potassium carbonate and 501 g of 3-methoxy-1-butanol (solvent 2) are added to the same reaction instrument as Example 1, heated to 95 to 105° C reacted for 2 hours maintaining 5 atm of $CO_2$. The reaction mixture is filtered at 95 to 105° C, and the filtrate is used in Example 3. The filtered precipitate is dissolved in 400 g of water, and neutralized by 190 g of concentrated. The white solid thus obtained is 128.7 g (0.84 mole, yield 87.4%) of 2,4-dihydroxybenzoic acid, having a melting point of from 224 to 225° C.

Example 3

92.5g (0.84 mole) of resorcinol and 200.2 g (1.45 mole) of potassium carbonate are added to the filtrate obtained in Example 2 and reacted together at 95-105° C, for 2 hours maintaining 5 atm of $CO_2$. The reaction mixtures are filtered at this temperatures, the filtered precipitates are dissolved in 380 g of water, and neutralized by 180 g of concentrated HCl. The white solid thus obtained is 125.2 g (0.81 mole, yield 96.7%) of 2,4-dihydroxybenzoic acid, having a melting point of from 224 to 226° c.

Example 4

165.2g (1.50 mole) of resorcinol, 100g (2.50 mole) of sodium hydroxide and 483g of 3-methoxy-1-butanol are added to the same reaction instrument as Example 1 and reacted together at a temperature of between 140 and 150° C for 3 hours maintaining 3 atm of $CO_2$. The reaction mixture is filtered at 130 to 140° C, the filtered precipitates is dissolved in 550 g of water, and neutralised by 250g of concentrated HCl. The white solid thus obtained is 220.5g (1.43 mole, yield 95.4%) of 2,4-dihydroxybenzoic acid, having a melting point of from 225 to 226° C.

Example 5

132.lg (1.20 mole) of resorcinol, 305.2g (2.88 mole) of sodium carbonate and 540g of ethyleneglycol monomethylether (solvent 3) are added to the same reaction instrument as Example 1 and reacted together at a temperature of between 120 and 125° C for 4 hours maintaining 6 atm of $CO_2$, the reaction mixture is filtered. The filtered precipitate is dissolved in 600g of water, and neutralized by 220g of concentrated HCl. The white solid thus obtained is 151.5g (0.98 mole, yield 81.9%) of 2,4-dihydroxybenzoic acid having a melting point of from 224 to 225° C.

Example 6

147.1g (1.02 mole) of 1-naphtol, 121.1g (1.14 mole) of sodium carbonate and 553 g of 1-methoxy-2-propanol are added to the same reaction instrument as Example 1, reacted together at a temperature of between 105 and 115° C for 5 hours maintaining 1 atm of $CO_2$, the reaction mixture is filtered. The filtered precipitate is dissolved in 530g of water, and neutralized by 170g of concentrated HCl. The white solid thus obtained is 176.6g (0.94 mole, yield 92.0%) of 1-hydroxy-2-naphtoic acid having a melting point of from 204 to 205° C.

Example 7

206.7g (1.88 mole) of rezorcinol, 337.3 g (2.44 mole) of potassium carbonate and 530 g of 1-methoxy-2-propanol are added to the same reaction instrument as Example 1, reacted at a temperature of between 105 and 115° C for 3 hours maintaining 3 atm of $CO_2$, the reaction mixture is filtered. The filtered precipitate is dissolved in 800g of water, and neutralized by 240g of concentrated HCl. The white solid thus obtained is 246.3g (1.60 mole, yield 85.0%) of 2,4-dihydroxybenzoic acid having an melting point of from 224 to

225°C.

Example 8

151.8g (1.38 mole) of resorcinol, 389.1g (2.82 mole) of potassium carbonate and 490g of ethylene glycol monomethylether are added to the same reaction instrument as Example 1, reacted at a temperature of between 110 and 120°C for 2 hours maintaining 4 atm of $CO_2$, and the reaction mixture is filtered. The filtered precipitate is dissolved in 750g of water and neutralized by 250g of concentrated HCl. The white solid thus obtained is 189.7g (1.23 mole, yield 89.2%) of 2,4-dihydroxybenzoic acid having a melting point of from 224 to 226°C.

## Claims

1. A process for the preparation of an aromatic carboxylic acid or a derivative thereof, characterized by dissolving a derivative of a hydroxylated aromatic compound in organic solvent chosen from those represented by formula (I) or (II) or mixtures thereof in the presence of $CO_2$ or a source of $CO_2$ in an autoclave:

$$R - X - (CH_2)n - Y \qquad (I)$$

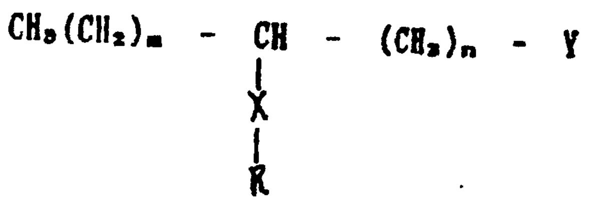

(II)

wherein, R is an alkyl group having tone to 4 carbon atoms.
X is -O-, -NH-,-S-, or

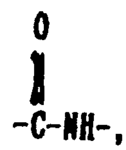

Y is -OH-, $-NH_2$ or

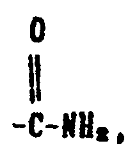

and
n is an integer from 1 to 4,
m is an integer from 0 to 3.

2. A process according to claim 1, wherein the organic solvent is chosen from ethyleneglycol monom ethyl ether, 3-methoxy-1-butanol or 1-methoxy-2-propanol.

3. A process according to claims 1 or 2, wherein the derivatives of hydroxylated aromatic compound is

chosen from derivatives of phenol, resorcinol or naphthol.

4. A process according to any preceding claim wherein the derivative of the hydroxylated organic compound and of the aromatic carboxylic acid is an alkaline salt.

5. A process according to claim 4 in which the salt of the aromatic carboxylic acid is neutralized to give the aromatic carboxylic acid.

6. A process for the preparation of an aromatic carboxylic acid, characterized in that it comprises the steps of
(i) reacting an alkali salt of a hydroxylated aromatic compound in an organic solvent chosen from those represented by formula (I) or (II) or mixtures thereof in an autoclave

$$R - X - (CH_2)n - Y \quad (I)$$

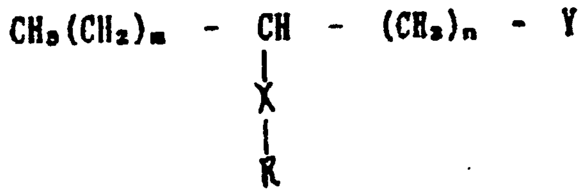

(II)

wherein, R is an alkyl group having one to 4 carbon atoms,
X is -O-, -NH-, -S-, or

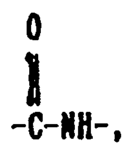

Y is -OH-, -$NH_2$or

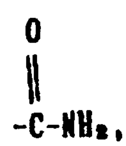

and
n is an integer from 1 to 4,
m is an integer from 0 to 3.
to form an alkali salt of an aromatic carboxylic acid, and
(ii) neutralising the alkali salt of the aromatic carboxylic acid to obtain the aromatic carboxylic acid.

7. A process according to claim 6 in which the alkali salt of the hydroxylated aromatic compound is formed in the organic solvent from the hydroxylated aromatic compound and a salt of an alkali metal.